(19) Europäisches Patentamt
European Patent Office
Office européen des brevets

(11) EP 1 029 063 B1

(12) **FASCICULE DE BREVET EUROPEEN**

(45) Date de publication et mention de la délivrance du brevet:
**09.09.2009 Bulletin 2009/37**

(21) Numéro de dépôt: **98954502.5**

(22) Date de dépôt: **02.11.1998**

(51) Int Cl.:
***C12N 15/74*** (2006.01) ***C12N 1/21*** (2006.01)

(86) Numéro de dépôt international:
**PCT/FR1998/002341**

(87) Numéro de publication internationale:
**WO 1999/024591 (20.05.1999 Gazette 1999/20)**

(54) **PLASMIDE DE LEUCONOSTOC NON RCR APTE A ETRE TRANSFERE DANS DES BACTERIES LACTIQUES; UTILISATION COMME OUTIL DE CLONAGE ET D'EXPRESSION**

NICHT RCR ,LEUCONOSTOCPLASMID TRANSFERFÄHIG IN MILCHSÄUREBAKTERIEN ; ANWENDUNG ALS KLONIERUNGS- UND EXPRESSIONSWERKZEUG

NON RCR LEUCONOSTOC PLASMID CAPABLE OF BEING TRANSFERRED INTO LACTIC ACID BACTERIA; USE AS CLONING AND EXPRESSING TOOL

(84) Etats contractants désignés:
**AT BE CH CY DE DK ES FI FR GB GR IE IT LI LU MC NL PT SE**

(30) Priorité: **06.11.1997 FR 9713977**

(43) Date de publication de la demande:
**23.08.2000 Bulletin 2000/34**

(73) Titulaire: **DANISCO A/S**
**1411 Copenhagen K. (DK)**

(72) Inventeurs:
- **BIET, Franck**
  **F-14460 Colombelle (FR)**
- **CENATIEMPO, Yves**
  **F-86800 Saint Julien l'Ars (FR)**
- **FREMAUX, Christophe**
  **F-86000 Poitiers (FR)**

(74) Mandataire: **Nargolwalla, Cyra et al**
**Cabinet Plasseraud**
**52, rue de la Victoire**
**75440 Paris Cedex 09 (FR)**

(56) Documents cités:
**WO-A-94/06917** **WO-A-94/16086**

- **BENACHOUR A ET AL: "pUCL287 plasmid from Tetragenococcus halophila (Pediococcus halophilus) ATCC 33315 represents a new theta-type replicon family of lactic acid bacteria." FEMS MICROBIOL LETT, MAY 1 1995, 128 (2) P167-75, XP002069942 NETHERLANDS**
- **FRERE J ET AL: "Molecular analysis of the Lactococcus lactis subspecies lactis CNRZ270 bidirectional theta replicating lactose plasmid pUCL22." MOL MICROBIOL, DEC 1993, 10 (5) P1113-24, XP002069943 ENGLAND cité dans la demande**
- **KIEWIET R ET AL: "Theta replication of the lactococcal plasmid pWVO2." MOL MICROBIOL, OCT 1993, 10 (2) P319-27, XP002069944 ENGLAND cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un plasmide utile notamment pour transférer et exprimer un gène hétérologue dans une bactérie, en particulier une bactérie lactique.

Les plasmides des bactéries lactiques peuvent être répartis en deux groupes suivant leur mode de réplication, soit un mode de réplication selon le modèle du cercle roulant (RCR), soit un mode de réplication similaire à celui du chromosome (Theta). Suivant le mode de réplication du plasmide, des éléments génétiques obligatoires ou non sont retrouvés dans la séquence nucléique du plasmide. Il peut s'agir de gènes directement impliqués dans la réplication du plasmide, de séquences nucléiques particulières, et de séquences nucléiques formant des structures particulières.

A ce jour, de nombreux vecteurs de clonage et d'expression développés pour les bactéries lactiques ont pour base des plasmides RCR isolées de bactéries lactiques (Leenhouts et al., 1991). Leur avantage principal réside dans leur large spectre d'hôte, cependant leur caractère instable nuit à leur utilisation.

Des plasmides théta issus de bactéries lactiques ont été décrits (Hayes et al., 1990 ; Kiewiet et al., 1993, Frère et al., 1993). Ils présentent notamment une bonne stabilité et la possibilité de transfert d'inserts de taille importante. Leur inconvénient majeur est toutefois un spectre d'hôte étroit. De ce fait, les vecteurs de type théta à spectre d'hôte large utilisés pour le clonage et l'expression de gènes hétérologues chez les bactéries lactiques ont été construits à partir de plasmides isolés d'entérocoques (Simon et Chopin, 1988) ou de staphylocoques (Le Chatelier et al., 1993). Leur utilisation agro-alimentaire est cependant difficilement envisageable en raison notamment de leur manque d'innocuité.

Un plasmide cryptique pUCL287, a été isolé de Tetragenococcus halophila (Pediococcus halophilus) ATCC 33315 (Benachour et al., 1995). Dans son hôte naturel, il avait un mécanisme de réplication de type theta. Son minimum réplicon, *Rep287,* a été isolé sur un fragment EcoRI de 1,6 kb. Le genre de l'hôte *Rep287* inclut les genres Pediococcus, Enterococcus, Lactobacillus et Leuconostoc, mais pas le genre Lactococcus.

Les auteurs de la présente invention ont à présent isolé un plasmide, référencé pTXL1, contenu dans des souches de bactéries lactiques, respectivement souches *Leuconostoc mesenteroïdes* Y110 et *Leuconostoc mesenteroïdes* Y111, dont il est établi que le mode de réplication n'est pas du type RCR, qui présente une stabilité relevée et un large spectre d'hôte au sein des bactéries lactiques.

Des plasmides possédant ces propriétés sont nouveaux et décrits pour la première fois dans le cadre de la présente invention.

L'invention a donc pour objet un plasmide selon les revendications dont le mode de réplication n'est pas du type RCR, et qui peut être transféré de façon stable dans des bactéries lactiques appartenant au moins à trois genres différents.

Avantageusement, le plasmide est issu de bactéries lactiques, notamment de bactéries lactiques du genre *Leuconostoc* en particulier *Leuconostoc mesenteroïdes.*

Par plasmide qui peut être transféré de façon stable dans des bactéries lactiques *Leuconostoc mesenteroïdes,* on entend au sens de la présente invention que le plasmide est maintenu dans la cellule hôte dans laquelle il a été transféré, en l'absence de pression de sélection, après 75 générations, de préférence après 90 générations, et plus particulièrement après 100 générations avec une taille apparente égale à la taille du plasmide natif, à l'exception des inserts qu'il contient éventuellement, y compris lorsqu'il est modifié par insertion d'une séquence d'une taille aussi importante que 4 kb., dès lors que l'insertion n'est pas réalisée dans les parties du plasmide nécessaires à sa réplication.

Les plasmides selon l'invention peuvent avantageusement être transférés de manière stable dans les bactéries lactiques appartenant aux genres *Leuconostoc, Lactobacillus, Pediococcus* et *Enterococcus,* notamment les espèces suivantes *Leuconostoc mesenteroïdes, Lactobacillus sake* et *Pediococcus acidilactici.*

Les inventeurs auteurs de la présente invention ont par ailleurs séquencé le plasmide pTXL1 et analysé sa séquence nucléique.

Ils ont localisé et déterminé un fragment de séquence ci-après dénommée SEQ ID N° 1 d'une longueur de 1346 pb, contenant la totalité des éléments requis pour la réplication autonome du plasmide. Cette séquence a été dénommée "minimum réplicon".

L'invention a donc également pour objet un plasmide tel que défini précédemment, comprenant la séquence nucléotidique SEQ ID N° 1 ou une séquence différant de celle-ci par insertion, délétion ou mutation d'une paire de base et qui conserve l'aptitude à se répliquer.

La séquence nucléique totale du plasmide pTXL1 a été déterminée et est dénommée séquence SEQ ID N° 2.

L'invention a donc également pour objet un plasmide tel que défini précédemment, comprenant la séquence nucléotidique SEQ ID N° 2 ou une séquence différant de celle-ci par insertion, délétion ou mutation d'une paire de base et qui conserve l'aptitude du plasmide à se répliquer de manière stable dans des cellules hôtes bactériennes de type lactique appartenant à au moins trois genres différents.

Deux souches de bactéries lactiques différentes contenant le plasmide ont été déposées dans une collection de microorganismes.

La souche Y110 a ainsi été déposée à la Collection Nationale de Culture de Microorganismes (CNCM) le 30 octobre 1997 sous le numéro I-1936.

La souche Y111 a ainsi été déposée à la Collection Nationale de Culture de Microorganismes (CNCM) le 30 octobre 1997 sous le numéro I-1937.

Les plasmides comprenant le cas échéant une séquence hétérologue insérée dans l'ADN plasmidique sont transférés dans les cellules hôtes à l'aide de techniques connues.

On peut citer notamment la technique de l'électroporation, en particulier celle mise au point pour la transformation des bactéries lactiques, notamment *Leuconostoc, Lactobacillus* et *Pediococcus.*

L'invention a également pour objet des cellules hôtes bactériennes abritant un plasmide selon l'invention, notamment les souches *Leuconostoc mesenteroïdes* Y110 et Y111 abritant le plasmide pTXL1.

En raison de la largeur de spectre d'hôte, les plasmides selon l'invention constituent des outils remarquables pour le clonage et l'expression de séquences nucléotidiques hétérologues dans des bactéries lactiques hôtes.

En particulier les plasmides selon l'invention peuvent être utilisés pour exprimer dans des cellules hôtes, notamment des bactéries lactiques, des protéines hétérologues, telles que la nisine, la mésentéricine Y105 qui sont des bactériocines, les protéines de résistance à ces bactériocines, appelées également protéines d'immunité et/ou une protéine de résistance à un antibiotique, par exemple l'érythromycine.

L'expression de protéines hétérologues du type mentionné ci-dessus est particulièrement intéressante, dans la mesure où elle permet, dans le cas où la protéine hétérologue est une bactériocine, de faire sécréter par une bactérie lactique présente dans un milieu pour une fonction donnée et présentant une innocuité totale, une substance capable d'empêcher la croissance d'autres bactéries non souhaitables voire pathogènes, notamment les *Listeria* qui ont tendance à croître dans les milieux lactiques.

Lorsque la protéine hétérologue est la protéine d'immunité à une bactériocine, l'expression de celle-ci a pour utilité de conférer à la bactérie lactique hôte la résistance à la bactériocine tout en empêchant la croissance de bactéries nuisibles ou pathogènes non souhaitées.

De même, lorsque la protéine hétérologue est une protéine de résistance à un antibiotique, il est alors possible d'ajouter l'antibiotique en question au milieu dans lequel se trouve la bactérie hôte sans nuire à la croissance et au métabolisme de celle-ci tout en agissant sur la croissance de microorganismes non souhaités.

L'invention a donc aussi pour objet l'utilisation d'un plasmide tel que décrit ci-dessus comme outil de clonage et d'expression de séquences nucléiques hétérologues dans des cellules hôtes, notamment des bactéries lactiques, en particulier les bactéries lactiques mentionnées ci-dessus.

L'invention a ainsi pour objet également un plasmide modifié obtenu à partir d'un plasmide tel que décrit précédemment par insertion d'une séquence nucléique hétérologue, de préférence une séquence nucléique codant pour un peptide ou une protéine hétérologue.

La séquence nucléique d'intérêt peut être insérée à l'aide des outils de biologie moléculaire appropriés, notamment d'enzymes de restrictions, ligase, etc. en un site quelconque de la séquence nucléique du plasmide, notamment de la séquence SEQ ID N° 2 à l'exception de la séquence SEQ ID N° 1.

La séquence nucléique codant pour le peptide ou la protéine hétérologue peut avoir une longueur jusqu'à au moins 4 kb, voire être plus importante.

L'invention a également pour objet les cellules hôtes contenant un plasmide modifié selon l'invention, notamment un plasmide comportant la séquence SEQ ID N° 1 et tout ou partie de la séquence SEQ ID N° 2 dans laquelle est insérée une séquence nucléotidique hétérologue.

Ces cellules hôtes sont notamment des bactéries lactiques, plus particulièrement appartenant aux genres *Leuconostoc, Lactobacillus* et *Pediococcus,* de préférence aux espèces *Leuconostoc mesenteroïdes, Lactobacillus sake* et *Pediococcus acidilactici.*

On décrira ci-après le clonage du plasmide pTXL1 et l'analyse de sa séquence ainsi qu'un plasmide recombinant construit à partir de pTXL1, en se référant aux figures 1 à 4 dans lesquelles :

- La figure 1 illustre la construction du plasmide pFBYC50E. Dans une première étape, pTXL1 a été linéarisé par l'enzyme SalI pour réaliser la fusion avec le plasmide pBSSKII+ linéarisé également au site SalI. Dans une deuxième étape, le gène de résistance à l'érythromycine EryR isolé du plasmide pGhost9IS*S1* a été inséré au site XbaI pour donner la construction pFBYC050E. Dans une dernière étape, une délétion PvuII sur la construction pFBYC050E élimine environ 85% de l'ADN d'origine pBSSKII+ pour aboutir au vecteur de clonage pFBYC50E.
- La figure 2 représente la carte de restriction partielle de pTXL1. Les étoiles indiquent une région de forte homologie de séquence avec des plasmides RCR. Les barres grisées indiquent les fragments de pTXL1 introduits dans le vecteur pBSSKII+ simultanément au gène de résistance à l'érythromycine. Le nom des plasmides ainsi obtenus est indiqué à gauche de la figure. A droite de la figure est indiqué la capacité (+) ou non (-) des plasmides à se répliquer dans la souche *Leuconostoc mesenteroides* DSM20484.
- La figure 3 représente la séquence nucléique du fragment SalI/SspI constituant le " minimum réplicon " de pTXL1.

Les sites de restrictions utilisés pour les constructions présentées à la figure 2 sont indiqués. La partie délétée dans le plasmide pFBYC065 est comprise entre le site SalI et la flèche verticale (position 314). Les séquences répétées inverses (IR flèches pleines) et répétées (DR flèches pointillées) sont indiquées au dessus de la séquence. La traduction des cadres ouverts de lectures ORF1 et ORF2 est présentée sous la séquence.

- La figure 4 illustre l'organisation génétique du fragment d'ADN cloné dans pFBYC051E permettant de sécréter la mésentéricine Y105. Ce fragment d'ADN est issu du plasmide pFBYCO7 décrit par Biet *et al.*(1997). Il comporte de gauche à droite :

  - un fragment *SmaI*/*PstI* de 551 pb issu du pGKV259 (Van Der Vossen *et al.*(1987)).
  - un fragment *EcoRVIHindIII* de 118 pb issu de pCD3.4 (Worobo *et al.* (1995)).
  - un fragment *HindIII* obtenu par PCR sur le plasmide pHY30 (Fremaux et al.(1995)).

P59 : promoteur p59 de *L.lactis* (Van Der Vossen *et al.*, 1987)) ; RBS : site de fixation du ribosome ; Ps : fragment du gène codant le peptide signal de la divergicine A (Worobo *et al.*(1995)) ; mesY : partie du gène codant la forme mature de la mésentéricine Y105, mesI : gène codant la protéine d'immunité à la mésentéricine.

Les souches utilisées dans cette études sont répertoriées dans le Tableau I. Leur propagation a été réalisée selon les méthodologies classiques de microbiologie dans les milieux de cultures et aux températures recommandées pour chaque espèce. Lorsque cela était nécessaire, de l'érythromycine ou de l'ampicilline étaient ajoutées aux milieux de culture, à raison de 5 μg/ml d'érythromycine pour les culture de bactéries lactiques et de 150 μg/ml d'érythromycine et de 75 μg/ml d'ampicilline pour les cultures *d'Escherichia Coli.*

Les plasmides caractérisés, utilisés et construits sont décrits dans le tableau II.

Les techniques générales de biologie moléculaire utilisées ont été décrites par Sambrook et al. (1989). Les enzymes de restriction et de modification ont été employées selon les recommandations du fournisseur (GIBCO-BRL). Les protocoles d'électroporation mis en oeuvre pour la transformation de *Lactobacillus* et *Pediococcus* sont décrits respectivement par Berthier et al. (1996) et Raya et al. (1992). La technique de Raya et al. (1992) a été utilisée pour la transformation de *Leuconostoc,* dans ce cas les bactéries sont préalablement cultivées en présence de 0,6 g/l de DL-thréonine. Pour la préparation du plasmide pTXL1, un extrait plasmidique de la souche *Leuconostoc mesenteroides* Y110 a été obtenu par la méthode décrite par Muriana et Klaenhammer (1991) puis purifié à partir d'un gel d'agarose de type ®SeaPlaque GTG (FCM BioProducts) à l'aide du kit ®Prep-a-Gene (Bio-Rad). L'analyse informatique des séquences nucléiques et protéiques déterminées a été réalisée à l'aide du logiciel ®GCG (USA).

La détection de l'activité antagoniste contre *L. ivanovii* a été réalisée par la méthode des "tests en goutte". Une gélose molle (0,6% d'agar) de milieu BHI (Difco), ensemencée à 0,2% par une culture en phase exponentielle de croissance de *L. ivanovii* CLIP257, est utilisée pour recouvrir un milieu BHI contenant 1,5% d'agar. Cinq μl d'un surnageant d'une culture sont ensuite adsorbés à la surface de la gélose "molle", après solidification. Les résultats sont lus après 18 heures d'incubation à 30°C. Pour tester la nature protéique des antagonistes détectés, 5μl d'une solution à 10 mg/ml de ®Protéinase K (Sigma) sont adsorbés à environ 2 cm du dépôt de surnageant de culture. La présence d'un antagoniste se traduit par l'apparition d'un halo (forme circulaire) d'inhibition de croissance. Ce halo d'inhibition sera partiellement détruit (forme de croissant) en présence de protéinase K, si l'antagoniste est de nature protéique. La molécule active produite par le clone DSM20484(pFBYC070) a été purifiée selon la méthode décrite par Biet *et al.,* (1997). Sa masse moléculaire a ensuite été déterminée par une analyse en spectrométrie de masse (Perkin Elmer SCIEX API 165).

**1. Mise en évidence du plasmide pTXL1**

La souche Y110 a été isolée à partir de laits de chèvres non-thermisés, au laboratoire (IBMIG, Université de Poitiers), et retenue pour sa propriété antagoniste vis à vis de *Listeria ssp.* Sa taxonomie a été déterminée par le "Deutsche Sammlung von Mikroorganismen" (DSM, Allemagne) ; il s'agit d'un *Leuconostoc mesenteroides* sous-espèce *mesenteroides.* Ses caractéristiques biochimiques sont rapportées dans le tableau III.

La présence d'ADN extra-chromosomique (ou plasmide) dans la souche Y110 a été déterminée. Celle-ci contient cinq plasmides caractéristiques de la souche. Le plasmide possédant une taille approximative de 2,6 kpb, dénommé pTXL1, fait l'objet de la suite de l'étude.

**2. Clonage du plasmide pTXL1**

La procédure est illustrée à la figure 1.

Les outils de biologie moléculaire ne sont pas adaptés à une analyse directe dans *Ln. mesenteroides.* L'analyse moléculaire du plasmide pTXL1 nécessite donc au préalable son clonage chez *E. coli,* une espèce utilisée en routine pour ce type d'analyse. Elle implique la séparation du plasmide pTXL1 des autres plasmides contenus dans la souche Y110, et la fusion du plasmide pTXL1 et du vecteur de clonage pBluescript II SK+ (pBSSKII+, Stratagene).

Les plasmides de la souche Y110 ont été séparés par électrophorèse sur gel à 0,8% d'agarose ®SeaPlaque GTG. La portion du gel contenant le plasmide pTXL1 a été isolée et le plasmide a été purifié.

La fusion du plasmide pTXL1 et du vecteur de clonage pBSSKII+ nécessite une étude préalable pour définir des sites uniques de restriction communs à ces deux plasmides. Les sites de restriction de pBSSKII+ sont décrits dans la littérature (Stratagene). Afin de rechercher les sites uniques de restriction de pTXL1, une portion du plasmide pTXL1, obtenu comme décrit ci-dessus, est soumis à l'action de diverses enzymes de restriction. Un site unique de restriction pour les enzymes de restriction *Sal*I, *Spe*I et *Ssp*I a pu être défini dans pTXL1 (Figure 1). Le plasmide pTXL1 et le vecteur de clonage pBSSKII+ ont été linéarisés par l'enzyme de restriction *Sal*I puis purifiés à l'aide du kit de purification d'ADN ®Prep-A-Gene. Les deux plasmides ainsi linéarisés ont ensuite été ligaturés par action de la T4 DNA polymérase, puis introduits dans la souche *E. coli* DH5$\alpha$. Le plasmide résultant de la fusion pTXL1/pBSSKII+ a été nommé pFBYC050 et présente une taille de 5,6 kb.

**3. Caractérisation moléculaire du plasmide pTXL1.**

- Détermination de la séquence nucléique.
  La séquence nucléique complète du plasmide pTXL1 a été déterminée à partir de la construction pFBYC050 par la méthode de Sanger et al. (1977) en utilisant le kit de séquençage ®AutoRead (Pharmacia) et un séquenceur automatique d'ADN (ALF, Pharmacia).
  La séquence a été dénommée séquence SEQ ID N° 2.
  La construction pFBYC051 a été réalisée selon la méthode décrite pour pFBYC050 mais en utilisant l'enzyme de restriction SpeI. Cette construction a été réalisée afin de vérifier la séquence du plasmide pTXL1 au site de restriction *Sal*I (en particulier pour s'assurer du clonage intégral du plasmide). De plus, la séquence nucléique complète du plasmide pTXL1 a été déterminée à nouveau à partir de la construction pFBYC051, permettant ainsi de confirmer la séquence obtenue initialement.
- Analyse de la séquence nucléique de pTXL1
  La séquence nucléique de pTXL1 a été comparée aux séquences nucléiques répertoriées dans les banques de données GenBank (USA) et EMBL (Allemagne). Une région d'environ 200 pb de pTXL1 présente une forte identité de séquence avec la famille des plasmides RCR de type pWV01 (Leenhouts et al., 1991). Le reste de la séquence ne présente pas d'identité avec les séquences contenues dans les banques de données.
  Cette homologie avec les plasmides de type pWV01 suggérait une réplication selon le modèle du cercle roulant (Gruss et Ehrlich, 1989). Cependant, des éléments essentiels à ce mode de réplication sont absents de pTXL1, en particulier, le gène codant la réplicase, un élément obligatoire à la réplication RCR. De plus, la recherche d'une forme simple brin du plasmide pTXL1 n'a jamais abouti, alors que ce type d'intermédiaire est en général détecté dans les cellules contenant un plasmide RCR. Finalement, les inventeurs ont montré par la suite que la séquence présentant des homologies avec les plasmide RCR n'était pas requise pour la réplication du plasmide (voir figure 2, plasmide FBY).
  L'analyse fine de la séquence, en particulier la recherche de cadres ouverts de lecture et de séquences inverses et directes répétées, a été réalisée sur le minimum réplicon, comme décrit plus bas.

**4. Construction d'un vecteur de clonage à partir de pTXL1.**

- Introduction d'un marqueur de sélection dans le plasmide pFBYC050.
  La transformation génétique des bactéries lactiques s'effectue par la méthode de l'électroporation (Bio-Rad). L'efficacité de cette transformation est très dépendante des souches utilisées. Elle varie classiquement de 10 à $10^6$ transformants par $\mu$g d'ADN en fonction de la souche réceptrice chez *L. lactis.* Après optimisation de la technique de transformation décrite par Raya et al. (1992), les inventeurs ont pu sélectionner une souche (*Ln. mesenteroides* DSM20484) présentant un degré de transformabilité élevé (approximativement $10^5$ transformants par $\mu$g d'ADN). Néanmoins, le taux de transformation des souches est toujours très faible. Par exemple, le taux de transformation de DSM20484 est inférieur à 1 bactérie transformée pour $10^5$ bactéries traitées. Il est donc absolument nécessaire d'introduire un marqueur de sélection des bactéries transformées lors de l'électroporation. Classiquement, des gènes de résistance aux antibiotiques sont utilisés comme marqueurs de sélection positive. Le gène de résistance à l'érythromycine issu du plasmide pAM$\beta$1 est connu pour sa fonctionnalité chez les bactéries lactiques et en particulier chez les leuconostocs. En conséquence, le gène codant la résistance à l'érythromycine (*ery*R) obtenu à partir du vecteur de clonage pGhost9IS*S1* (Maguin et al, 1996) a été introduit au site de restriction unique *Xba*I du pFBYC050 comme montré dans la figure 1. Cette construction, appelée pFBYC050E, a été obtenue préalablement chez *E. coli* puis transférée avec succès dans DSM20484.

- Elimination du réplicon pBSSKII+
  Le plasmide pFBYC050E est donc opérationnel dans la souche DSM20484. Cependant, pour pouvoir affirmer que la réplication de ce plasmide est assurée par les fonctions apportées par le plasmide pTXL1, il est nécessaire d'éliminer les fonctions de réplication portées par le pBSSKII+.
  Cette délétion a été réalisée par une double coupure par l'enzyme de restriction *Pvu*II comme illustré à la figure 1. Cette double coupure permet l'excision d'un fragment représentant plus de 85 % du pBSSKII+, y compris de la totalité de ses fonctions de réplication. Le plasmide, appelé pFBYC50E et résultant de cette manipulation, a été introduit avec succès dans la souche DSM20484, démontrant ainsi sans équivoque la fonctionnalité du réplicon sous cette configuration, qui peut être assimilée à un vecteur de clonage.
- Localisation du minimum réplicon.
  Afin de cerner au plus près les éléments codés par pTXL1 et requis pour sa réplication, divers fragments du plasmides ont été fusionnés avec le vecteur pBSSKII+. Ensuite, un gène marqueur (gène de résistance à l'érythromycine) a été introduit dans ces constructions. Ces plasmides ont été obtenus dans *E. coli* grâce aux fonctions de réplication apportées par pBSSKII+, et sont présentés dans le Tableau II et dans la figure 2. Les fonctions de réplication apportées par pBSSKII+ n'ont donc pas été éliminées, puisque les inventeurs ont démontré ci-dessus qu'elles ne jouent aucun rôle dans cette configuration.
  Par la suite, ces constructions ont été utilisées pour la transformation de DSM20484. Etant donné l'efficacité de transformation élevée obtenue pour cette souche (104 à 105 transformants par μg d'ADN), il a été considéré que l'intégralité des fonctions de réplication n'était pas présente dans le fragment de pTXL1 cloné, lorsqu'aucun transformant n'était obtenu. Les résultats obtenus illustrés à la figure 2 ont permis de définir un fragment minimum de 1346 pb, borné par les sites de restriction *Sal*I et *Ssp*I, contenant la totalité des éléments requis pour la réplication autonome de pTXL1. Ce fragment appelé minimum réplicon comprend la séquence SEQ ID N° 1.

**5. Analyse du minimum réplicon de pTXL1**

- Recherche de cadres ouverts de lecture (ORF)
  La littérature abonde d'exemples de plasmides isolés de bactéries lactiques codant des protéines, appelées réplicases, qui jouent un rôle essentiel dans la réplication de ces plasmides. Des ORF ont donc été recherchés dans le minimum réplicon de pTXL1. Deux ORF courts ont été trouvés (figure 3) : ORF1 (nucléotides 1000 à 1341) et ORF2 (nucléotides 883 à 640). Dans leur voisinage immédiat, des séquences ressemblant à des sites de fixation du ribosome (RBS) nécessaires à la traduction des ORF en protéines ont été détectées. Cependant, aucun codon d'initiation de la traduction n'est placé à la distance adéquate de ces RBS potentiels, suggérant que ces CRF pourraient ne pas être traduits. Les séquences protéiques ont néanmoins été déduites de ces ORF et ont été comparées aux séquences protéiques contenues dans les banques de données GenBank (USA) et SwissProt (EMBL, Allemagne). Elles ne présentent aucune similitude avec des protéines déjà décrites.
  Les ORF1 et ORF2 sont affectés par la coupure par *Afl*III réalisée pour l'obtention de pFBYC068E (figure 2). Cette construction n'est pas capable de se répliquer de façon autonome, il est donc possible que ces séquences jouent un rôle dans la réplication de pTXL1.
- Recherche de séquences répétées (DR) et répétées inverses (IR)
  Il est connu de par la littérature que des séquences DR et/ou IR sont impliquées dans la réplication de nombreux plasmides. Elles ont la particularité de pouvoir induire la formation de structures secondaires. Certaines d'entre elles sont décrites mais elles ne sont pas présentes dans pTXL1. Par contre, au sein du minimum réplicon de pTXL1, deux séquences IR (référencées IR1 et IR2, figure 3) ont été détectées. Elles sont susceptibles de générer dés structures secondaires dont les $\Delta G_0$ calculés sont de -10,6 et -22,4 kcal/mole. Il est a noter également la présence de 3 séquences DR référencées DR1, DR2, DR3 à la figure 3.
  Dans le plasmide pFBYC065 qui ne se réplique pas dans DSM20484, la région contenant les élément IR1 et DR1 est absente. Il est donc possible que ces séquences aient un rôle dans la réplication de pTXL1.

**6. Performance du plasmide pFBYC50E**

- Stabilité

La plupart des vecteurs de clonage de qualité alimentaire développés pour les bactéries lactiques sont issus de plasmides de type RCR et s'avèrent instables en absence de pression de sélection. Afin d'évaluer la stabilité du plasmide pFBYC50E, un clone de DSM20484 contenant pFBYC50E a été isolé sur milieu de culture MRS sélectif (contenant 10µg/ml d'érythromycine). Ce clone a ensuite servi à inoculer au 1‰ successivement des milieux MRS non-sélectifs (ne contenant pas d'érythromycine) de manière à multiplier le clone sur au moins 100 générations. La stabilité du plasmide est ensuite évaluée par la proportion de sous-clones ayant conservé le plasmide. Cette mesure est effectuée par comparaison d'étalement d'une dilution de la culture finale (après 100 générations) sur milieu sélectif et non-sélectif. La stabilité du plasmide est exprimée en pourcentage et correspond à :

$$\frac{\text{Nombre de sous-clones obtenus sur milieu sélectif}}{\text{Nombre de sous-clones obtenus sur milieu non-sélectif}} \times 100$$

Les résultats du Tableau IV montrent clairement la stabilité du plasmide pFBYC50E, comparée à celle du plasmide RCR pFR18.

- Spectre d'hôte
  Le spectre d'hôte des vecteurs développés à partir de plasmides de type Theta isolés de bactéries lactiques est généralement étroit. Des transformations avec le plasmide pFBYC50E ont été tentées sur des bactéries appartenant aux groupes lactiques principaux, les plus utilisés industriellement : *Lactobacillus, Pediococcus* et *Leuconostoc.* Les souches retenues ont été choisies en fonction de leur niveau de transformabilité (*Lactobacillus sake* 23K ; *Pediococcus acidilactici* PAC1.0 et *Leuconostoc mesenteroides* DSM20484) en utilisant les protocoles de transformation les mieux adaptés. Comme le montre le tableau V, des transformants ont pu être obtenus pour toutes les souches.
- Efficacité de transformation
  Le plasmide pFBYC50E et le plasmide pFBYC018E, un vecteur construit à partir d'un plasmide de type RCR issu de *Ln mesenteroides,* ont été utilisés pour évaluer l'efficacité de transformation par électroporation de la souche *Lb. sake* 23K. Ces deux plasmides possèdent le même marqueur de sélection et ont été préparés à partir de la même souche hôte. Des quantités équivalentes de plasmides et une même préparation de bactéries électro-compétentes ont été utilisées. A partir de trois expériences indépendantes, les rendements de transformation obtenus avec le plasmide pFBYC50E sont d'environ $10^4$ transformants par µg d'ADN, soit une efficacité approximativement 1000 fois supérieure à celle obtenue avec le vecteur construit à partir de pFR18.

**7. Utilisation du réplicon pour la production d'une bactériocine chez les bactéries lactiques**

Les bactériocines sont des peptides anti-bactériens. En particulier, la mésentéricine Y105 est une bactériocine, produite par *Ln mesenteroides* Y105 présentant une activité contre *Listeria.* Ses déterminants génétiques ont été décrits (Fremaux et al. 1995), ce qui a permit de montrer que sa production dans le milieu extra-cellulaire est dépendante d'un système spécifique de transport. Pour s'affranchir des problèmes potentiels liés à ce système de transport lors de l'expression hétérologue de la bactériocine, un ensemble génétique susceptible de sécréter la mésentéricine Y105 a été construit (Biet et al, 1997). En effet, la sécrétion constitue un système de transport universel chez les bactéries. Cet ensemble génétique a ensuite été introduit dans un plasmide dérivé de pTXL1.

• Sécrétion de la mésentéricine Y105.
Pour permettre la sécrétion de la mésentéricine Y105, plusieurs éléments génétiques, fonctionnels chez les bactéries lactiques, ont été associés comme décrit dans la figure 4 et par Biet et al. (1997). Ces éléments sont :

1. le promoteur constitutif p59 issu de *L lactis* (Van der Vossen et al, 1987) ;
2. le site de fixation du ribosome (RBS) localisé en amont du gène de structure de la divergicine A (*dvnA*) produite par *Carnobacterium divergens* LV13 (Worobo et al., 1995) ;
3. la partie du gène de structure de la divergicine A (*dvnA*) codant le peptide signal (Worobo et al., 1995) ;
4. la partie du gène de structure de la mésentéricine Y105 (*mesY*) codant la partie mature de la mésentéricine Y105 (Fremaux et al, 1995);
5. le gène *mesI* codant la protéine d'immunité à la mésentéricine Y105.

• Construction du plasmide permettant la sécrétion de la mésentéricine Y105

Construction de pFBYC051 E : La construction a été réalisée en introduisant un fragment *Bam*HI codant pour la résistance à l'érythromycine dans le plasmide pFBYC051, comme décrit dans la figure 1 pour pFBYC050E.

Construction de pFBYC070 : Le fragment d'ADN permettant la sécrétion de la mésentéricine Y105 (décrit dans la figure 4) a été introduit aux sites de restriction *Sma*I et *Hind*III du plasmide pFBYC051 E. Cette construction a été réalisée directement dans la souche *Ln. mesenteroides* DSM20484 étant donné la toxicité de la mésentéricine Y105 clonée chez *E. coli.*

• Expression de la mésentéricine Y105 par le clone de DSM20484 contenant le plasmide pFBYC070.

La présence d'un antagoniste actif contre *L. ivanovii* CLIP257 a pu être visualisée dans les surnageants de culture de la souche DSM20484 contenant pFBYCO70, par la méthode des tests en goutte. La nature protéique de la molécule active a été démontrée par sa sensibilité à la protéinase K. L'antagoniste a ensuite été purifié à 99 % par la méthode mise au point par et décrite par Biet et al. (1997) et sa masse moléculaire a été déterminée par spectrométrie. La valeur obtenue de 3868 $\pm$ 0,1 Da correspond exactement à la masse théorique calculée de la forme réduite de la mésentéricine Y105.

Tableau I:

Descriptif des souches bactériennes utilisées.

| Souches | Descriptif | Références |
|---|---|---|
| *Escherichia coli* | | |
| DH5$\alpha$ | *recA EndA1 gyrA96 thi-1 hsdR17 supE44* $\Delta$*lac* u169($\phi$80 dlacZ$\Delta$M15)*deo*RF-$\lambda$- | GIBCO-BRL |
| *Leuconostoc mesenteroides* ssp. *Mesenteroides* | | |
| Y110 | Souche sauvage | présente invention |
| Y111 | Souche sauvage | présente invention |
| Y105 | Souche sauvage | Fremaux *et al.*(1995) |
| FR52 | Souche sauvage | Mathieu *et al.* (1993) |
| *Leuconostoc mesenteroides* ssp. *Dextranicum* | | |
| DSM 20484 *Lactobacillus sake* | Souche sauvage | DSM |
| 23K | Souche sans plasmides | Berthier *et al.*(1996) |
| *Pediococcus acidilactici* | | |
| PAC 1.0 | Souche sauvage | Marugg *et al.*(1992) |
| *Listeria ivanovii* CLIP257 | Souche sauvage | Collection de l'Institut Pasteur |

Tableau II :

Descriptif des plasmides utilisés ou construits.

| Plasmides | Descriptif | Références |
|---|---|---|
| pTXL 1 | plasmide naturel de *Ln mesenteroides* Y110 | non publié |
| pBSSKII+ | pBluescript II SK+, vecteur de clonage *E. coli* | Stratagene |
| pGKV259 | Vecteur de clonage navette *E. coli*/bactéries lactiques | Van der Vossen *et al* .(1987) |
| pHY30 | plasmide naturel de *Ln Mesenteroides* Y105 | Fremaux *et al.* (1996) |
| pCD3.4 | plasmide naturel de *Carnobacterium divergences* LV13 | Worobo *et al* (1995) |
| pGhost91S*S1* | vecteur de clonage source de *ery*R | Maguin *et al.* (1996) |
| pFR18 | plasmide naturel de *Leuconostoc mesenteroides* ssp. *Mesenteroides* FR52 | Biet *et al.* (1997) |

(suite)

| Descriptif des plasmides utilisés ou construits. | | |
|---|---|---|
| **Plasmides** | **Descriptif** | **Références** |
| pFBYC018E | Dérivé de pFR18 | Biet *et al.* (1997) |
| pFBYC050 | insertion de pTXL1 linéarisé par -*Sal*I au site *Sal*I de pBSSKII+ | présente invention |
| pFBYC051 | insertion de pTXL1 linéarisé par *Spe*I au site *Spe*I de pBSSKII+ | présente invention |
| pFBYC052 | insertion de pTXL1 linéarisé par *Ssp*I au site *Sma*I de pBSSKII+ | présente invention |
| pFBYC050E | pFBYC050::ErmR | présente invention |
| pFBYC50E | pFBYC050E délété de pBSSKII par double restriction *Pvu*II | présente invention |
| pFBYC065 | pFBYC050E délété de 312 pb | présente invention |
| pFBYC060K | pFBYC050E délété *Kpn*I-*Kpn*I de 482 pb | présente invention |
| pFBYC062S | pFBYC052E délété *Sal*I-*Ssp*I de 1400 pb | présente invention |
| pFBYC068E | pFBYC050E délété *Sal*I-*Afl*III de 1893 pb | présente invention |
| pFBYC051E | pFBYC051::ErmR | présente invention |
| pFBYCO7 | vecteur de secrétion dérivé du pGKV259 et du pCD3.4 | Biet *et al.* (1997) |
| pFBYC069 | insertion dans pFBYC051E de la fusion p59/signa-*dvnA* | présente invention |
| pFBYC070 | insertion dans pFBYC069 des gènes mature-*mes Y* et *mesI* | présente invention |

Tableau III :

Description de la souche *Leuconostoc mesenteroides* subsp. *mesenteroides* Y110 par la "Deutsche Sammlung von Mikroorganismen" (DSM).

Coque à Gram-positif, apparaissant en paires ou en chaînettes, anaérobie facultatif ; produits de la fermentation du glucose : acide lactique, éthanol et $CO_2$.

| | | | |
|---|---|---|---|
| Mobilité | - | Croissance à 45°C | - |
| Formation de spores | - | Gaz à partir du glucose | + |
| Catalase | - | Gaz à partir de gluconate | - |
| Croissance à 15°C | + | | |
| Production d'acide à partir de : | | | |
| Ribose | + | Glucose | + |
| Arabinose | - | Lactose | - |
| Xylose | + | Maltose | + |
| Rhamnose | - | Sucrose | + |
| Manitol | - | Tréhalose | + |
| Sorbitol | - | Cellobiose | - |
| Ribitol | - | Raffinose | - |
| Glycérol | - | Mélibiose | - |
| Fructose | + | Mélézitose | - |
| Mannose | + | Salicine | - |
| Galactose | + | Gluconate | - |

$NH_3$ à partir d'arginine - Configuration de l'acide lactique : D(-) Aucun acide meso-diaminopimelique dans l'hydrolysat cellulaire.

Type de peptidoglycanne : A3$\alpha$, Lys-Ser-$Ala_2$

Tableau IV :

| Stabilité de dérivés du plasmide pTXL1 en absence de pression de sélection. | |
|---|---|
| **Plasmides** | **Stabilité*** |
| pFBYC050E | 100 |
| pFBYC50E | 100 |
| Plasmide RCR témoin (par18) | 3 |
| * pourcentage des clones isolés après 100 générations contenant le plasmide | |

Tableau V :

| Spectre d'hôte des dérivés de pTXL1 (pFBYC050E et/ou pFBYC50E) ont été utilisés. | |
|---|---|
| **Souches** | **Transformabilité** |
| *Leuconostoc mesenteroides* LM20484 | + |
| *Lactobacillus sake* 23K | + |
| *Pédiococcus acidilactici* PAC1.0 | + |

**BIBLIOGRAPHIE**

Benachour, A., J. Frère, and G. Novel. 1995. pUCL287 plasmid from Tetragenococcus halophila (Pediococcus halophilus) ATCC 33315 represents a new theta-type repicon family of lactic acid bacteria. FEMS Microbiology Letters. 128:167-176.

Berthier, F., M. Zagorec, M. Champomier-Vergès, S. D. Ehrlich, and F. Morel-Deville. 1996. Efficient transformation of Lactobacillus sake by electroporation. Microbiology. 142:1273-1279.

Biet, F., J.M. Berjeaud, R. W., Worobo, Y. Cenatiempo, and C. Fremaux 1997. Heterologous expression of mesentericin Y105 using the general secretion pathway (GST) or the dedicated transport sytem (DTS) in gram-positive and gram-negative bacteria.Soumission prochaine pour publication dans Microbiology.

**Biet F., Cenatiempo, and C. Fremaux** 1997, Nucleotide sequence analysis and characterization of pFR18 a small cryptic plasmid for *Leuconostoc mesneteroides* ssp. Mesenteroides Fr52 and its use as a vector. Soumis pour publication.

Fremaux, C., Y. Héchard, and Y. Cenatiempo. 1995. Mesentericin Y105 gene clusters in Leuconostos mesenteroides Y105. Microbiology. 141:1637-1645.

Frère, J., M. Novel, and G. Novel. 1993. Molecular analysis of the Lactococcus lactis subspecies lactis CNRZ270 bidirectional theta replicating lactose plasmid pUCL22. Molecular Microbiology. 10(5): 1113-1124.

Gruss, A., and D. Ehrlich. 1989. The family of interrelated single-stranded deoxyribonucleic acid plasmids. MICROBIOLOGICAL REVIEWS. june:231-241.

Hayes, F., C. Daly and G.F. Fitzgerald. 1990, Identification of the minimum replicon of Lactococcus lactis subsp. lactis UC317 plasmid pCl305. Applied and Environmental Microbiology 56(1):202-209.

Kiewiet, R., S. Bron, K. De Jonge, G. Venema, and J. F. M. L. Seegers. 1993. Theta replication of the lactococcal plasmid pWV02. Molecular Microbiology. 10(2):319-327.

Le Chatelier, E., D. S. Ehrlich, and L. Jannière. 1993. Biochemical and genetic analysis of the unidirectional theta replication of the S. agalactiae plasmid pIP501. PLASMID. 29: 50-56.

Leenhouts, K. J., B. Tolner, S. Bron, J. Kok, G. Venema, and J. F. M. L. Seegers. 1991. Nucleotide sequence and characterization of the Broad-host-range Lactococcal plasmid pWV01. PLASMID. 26: 55-66.

Maguin, E., H. Prévost, D. S. Ehrlich, and A. Gruss. 1996. Efficient insertioal mutagenesis in Lactococci and other Gram-positive bateria. Journal of bacteriology. 178(3):931-935.

Marugg, J. D., C. F. Gonzales, B. S. Kunka, A. M. Ledeboer, M. J. Pucci, M. Y. Toonen, S. A. Walker, L. C. Zoetmulder, and P. A. Vandenbergh. 1992. Cloning, expression, and nucleotide sequence of genes involved in production of PA-1, a bacteriocin from Pediococcus acidilactici PAC1.0. APPLIED AND ENVIRONMENTAL MICROBIOLOGY. 58(8):2360-2367.

Muriana, P. M., and T. R. Klaenhammer. 1991. Cloning, phenotypic expression and DNA sequence of the gene for lactacin F, an antimicrobial peptide produced by Lactobacillus spp. Journal of Bacteriology, 173(5):1779-1788.

Raya, R. R., C. Fremaux, G. L. De Antoni, and T. R. Klaenhammer. 1992. Site-specific integration of the temperate bacteriophage fadh into the Lactobacillus gasseri chromosome and molecular characterization of the phage (attP) and bacterial(attB) attachment site. Journal of Bacteriology. 174:5584-5592.

Sambrook, J., E. Fritsch, and T. Maniatis (ed.). 1989. Molecular cloning:a laboratory manual, NY : Cold sping harbor laboratory.

Sanger, F., S. Nicklen, and A. R. Coulson. 1977. DNA sequencing with chain-terminating inhibitions. Proc. Natl. Acad Sci. 74:5463-5467.

Simon, D., and A. Chopin. 1988. Construction of a vector plasmid family and its use for molecular cloning in Streptococcus lactis. Biochimie. 70: 559-566.

Van Der Vossen, J. M. B. M., D. Van Der Lelie, and G. Venema. 1987. Isolation and characterization of Streptococcus cremoris Wg2-specific promoters. Applied and Environmental Microbiology. 53(10): 2452-2457.

Wororb, R. W., M. J. Van Belkum, M. Sailer, K. L. Roy, J. C. Vederas, and M. E. Stiles. 1995. A signal peptide secretion-dependent bacteriocin from Carnobacterium divregens. Journal of Bacteriology. 177(11):3143-3149.

## Revendications

1. Plasmide **caractérisé en ce qu'**il comprend la séquence nucléotidique SEQ ID NO:1.

2. Plasmide **caractérisé en ce qu'**il comprend une séquence différant de la séquence telle que définie dans la revendication 1 par insertion délétion ou mutation d'une paire de base et qui conserve l'aptitude du plasmide à se répliquer de manière stable et selon un mode non RCR, dans des cellules hôtes bactériennes de type lactique appartenant à au moins trois genres différents.

3. Plasmide selon la revendication 1 ou 2, **caractérisé en ce qu'**il comprend la séquence nucléotidique SEQ ID NO:2.

4. Plasmide **caractérisé en ce qu'**il comprend une séquence différant de la séquence telle que définie dans la revendication 3 par insertion, délétion ou mutation d'une paire de base et qui conserve l'aptitude du plasmide à se répliquer de manière stable et selon un mode non RCR, dans des cellules hôtes bactériennes de type lactique appartenant à au moins trois genres différents.

5. Plasmide selon l'une quelconque des revendications précédentes ayant un mode de réplication qui n'est pas du type RCR, et pouvant être transféré de façon stable dans des bactéries hôtes lactiques appartenant au moins à trois genres différents, ledit plasmide étant issu d'une bactérie lactique du genre *Leuconostoc.*

6. Plasmide selon la revendication 5, ledit plasmide étant issu d'une bactérie lactique *Leuconostoc mesenteroïdes.*

7. Plasmide selon la revendication 5 ou 6, **caractérisé en ce qu'**il est stable dans des cellules hôtes après 75, de préférence après environ 90 générations, de préférence après environ 100 générations, en l'absence de pression de sélection.

8. Plasmide selon l'une quelconque des revendications 5 à 7, **caractérisé en ce que** les bactéries hôtes appartiennent au moins aux genres *Leuconostoc, Lactobacillus* et *Pediococcus.*

9. Plasmide selon la revendication 8, **caractérisé en ce que** les bactéries lactiques hôtes appartiennent aux espèces *Leuconostoc mesenteroïdes, Lactobacillus sake* et *Pediococcus acidilactici.*

10. Utilisation d'un plasmide selon l'une des revendications précédentes, comme outil de clonage et d'expression de séquences nucléotidiques hétérologues.

11. Plasmide modifié obtenu à partir d'un plasmide tel que défini à l'une quelconque des revendications 1 à 9 par insertion dans ledit plasmide d'une séquence nucléique hétérologue, de préférence une séquence nucléique codant pour un peptide ou une protéine hétérologue.

12. Plasmide selon la revendication 11, **caractérisé en ce qu'**il comprend une séquence nucléique choisie parmi une séquence nucléique codant pour une bactériocine, une séquence nucléique codant pour une protéine d'immunité à une bactériocine et une séquence nucléique codant pour une protéine de résistance à un antibiotique.

13. Plasmide modifié selon la revendication 11 ou la revendication 12, **caractérisé en ce qu'**il comprend la séquence SEQ ID NO:1 ainsi qu'une séquence nucléotidique hétérologue insérée dans le génome du plasmide en un site autre que la séquence SEQ ID NO:1.

14. Plasmide modifié selon la revendication 11 ou la revendication 12, **caractérisé en ce que** la séquence nucléotidique hétérologue est insérée en un site quelconque de la séquence SEQ ID NO:2 à l'exception de la séquence SEQ ID NO:1.

15. Cellule hôte transformée par un plasmide selon l'une des revendications 5 à 9, ou un plasmide modifié selon l'une quelconque des revendications 12 à 14, ladite cellule ne comprenant pas naturellement ledit plasmide.

16. Cellule hôte comportant un plasmide comportant la séquence SEQ ID NO:1 et tout ou partie de la séquence SEQ ID NO:2 dans laquelle est insérée une séquence nucléotidique hétérologue.

17. *Leuconostoc mesenteroides* comprenant le plasmide pTLX1 déposé à la collection Nationale de Cultures de Microorganismes (CNCM) le 30 octobre 1997 sous le numéro I-1936.

18. *Leuconostoc mesenteroides* comprenant le plasmide pTLX1 déposé à la collection Nationale de Cultures de Microorganismes (CNCM) le 30 octobre 1997 sous le numéro I-1937.

## Claims

1. Plasmid, **characterised in that** it comprises the nucleotide sequence SEQ ID NO:1.

2. Plasmid, **characterised in that** it comprises a sequence which differs from the sequence as defined in claim 1 by insertion, deletion or mutation of a base pair and which retains the ability of the plasmid to replicate in a stable manner and in accordance with a non-RCR mode, in lactic acid-type host bacteria cells belonging to at least three different genera.

3. Plasmid according to claim 1 or 2, **characterised in that** it comprises the nucleotide sequence SEQ ID NO:2.

4. Plasmid, **characterised in that** it comprises a sequence which differs from the sequence as defined in claim 3 by insertion, deletion or mutation of a base pair and which retains the ability of the plasmid to replicate in a stable manner and in accordance with a non-RCR mode, in lactic acid-type host bacteria cells belonging to at least three different genera.

5. Plasmid according to any one of the preceding claims, having a replication mode which is not of the RCR type, and capable of being transferred in a stable manner into host lactic acid bacteria belonging at least to three different genera, said plasmid being derived from a lactic acid bacterium of the genus *Leuconostoc.*

6. Plasmid according to claim 5, said plasmid being derived from a *Leuconostoc mesenteroides* lactic acid bacterium.

7. Plasmid according to claim 5 or 6, **characterised in that** it is stable in host cells after 75, preferably after approximately 90 generations, preferably after approximately 100 generations, in the absence of selection pressure.

8. Plasmid according to any one of claims 5 to 7, **characterised in that** the host bacteria belong at least to the genera *Leuconostoc, Lactobacillus* and *Pediococcus.*

9. Plasmid according to claim 8, **characterised in that** the host lactic acid bacteria belong to the species *Leuconostoc mesenteroides, Lactobacillus sake* and *Pediococcus acidilactici.*

10. Use of a plasmid according to one of the preceding claims, as a tool for cloning and expressing heterologous nucleotide sequences.

11. Modified plasmid obtained from a plasmid as defined in any one of claims 1 to 9 by inserting into said plasmid a heterologous nucleic acid sequence, preferably a nucleic acid sequence coding for a heterologous peptide or protein.

12. Plasmid according to claim 11, **characterised in that** it comprises a nucleic acid sequence selected from a nucleic acid sequence coding for a bacteriocin, a nucleic acid sequence coding for a protein for immunity to a bacteriocin and a nucleic acid sequence coding for a protein for resistance to an antibiotic.

13. Modified plasmid according to claim 11 or claim 12, **characterised in that** it comprises the sequence SEQ ID NO:1 and also a heterologous nucleotide sequence inserted into the genome of the plasmid at a site other than the sequence SEQ ID NO:1.

14. Modified plasmid according to claim 11 or claim 12, **characterised in that** the heterologous nucleotide sequence is inserted at any desired site of the sequence SEQ ID NO:2 except for the sequence SEQ ID NO:1.

15. Host cell transformed by a plasmid according to one of claims 5 to 9, or a modified plasmid according to any one of claims 12 to 14, said cell not naturally comprising said plasmid.

16. Host cell comprising a plasmid comprising the sequence SEQ ID NO:1 and all or part of the sequence SEQ ID NO:

2 into which a heterologous nucleotide sequence is inserted.

**17.** *Leuconostoc mesenteroides* comprising the plasmid pTLX1 deposited in the Collection Nationale de Cultures de Microorganismes ("French National Collection of Microorganism Cultures") (CNCM) on 30 October 1997 under number I-1936.

**18.** *Leuconostoc mesenteroides* comprising the plasmid pTLX1 deposited in the Collection Nationale de Cultures de Microorganismes (CNCM) on 30 October 1997 under number I-1937.

**Patentansprüche**

**1.** Plasmid, **dadurch gekennzeichnet, dass** es die Nucleotidsequenz SEQ ID Nr. 1 umfasst.

**2.** Plasmid, **dadurch gekennzeichnet, dass** eine Sequenz umfasst, die von der Sequenz, die in Anspruch 1 definiert wurde, durch Insertion, Deletion oder Mutation eines Basenpaares verschieden ist und die Fähigkeit des Plasmids bewahrt, sich in stabiler Weise und nach einem Modus, der nicht RCR ist, in Milchsäurebakterien-Wirten zu replizieren, die zu mindestens drei verschiedenen Genera gehören.

**3.** Plasmid gemäß Anspruch 1 oder 2, **dadurch gekennzeichnet, dass** es die Nucleotidsequenz SEQ ID Nr. 2 umfasst.

**4.** Plasmid, **dadurch gekennzeichnet, dass** es eine Sequenz umfasst, die von der Sequenz, die in Anspruch 3 definiert wurde, durch Insertion, Deletion oder Mutation eines Basenpaares verschieden ist und die Fähigkeit des Plasmids bewahrt, sich in stabiler Weise und nach einem Modus, der nicht RCR ist, in Milchsäurebakterien-Wirten zu replizieren, die zu mindestens drei verschiedenen Genera gehören.

**5.** Plasmid gemäß irgendeinem der vorhergehenden Ansprüche mit einem Replikationsmodus, der nicht vom RCR-Typ ist, und welches in stabiler Weise in Milchsäurebakterien-Wirte überführt werden kann, die zu mindestens drei verschiedenen Genera gehören, wobei das Plasmid aus einem Milchsäurebakterium von Genus Leuconostoc stammt.

**6.** Plasmid gemäß Anspruch 5, wobei das Plasmid aus einem Leuconostoc mesenteroides Milchsäurebakterium stammt.

**7.** Plasmid gemäß Anspruch 5 oder 6, **dadurch gekennzeichnet, dass** es in Wirtszellen nach 75, vorzugsweise nach ungefähr 90, vorzugsweise nach ungefähr 100 Generationen ohne Selektionsdruck stabil ist.

**8.** Plasmid gemäß irgendeinem der Ansprüche 5 bis 7, **dadurch gekennzeichnet, dass** die Wirtszellen zu mindestens einem der Genera Leuconostoc, Lactobacillus und Pediococcus gehören.

**9.** Plasmid gemäß Anspruch 8, **dadurch gekennzeichnet, dass** die Milchsäurebakterien-Wirte zu den Arten Leuconostoc mesenteroides, Lactobacillus sake und Pediococcus acidilactici gehören.

**10.** Verwendung eines Plasmids gemäß irgendeinem der vorhergehenden Ansprüche als Mittel zur Klonierung und Expression von heterologen Nucleotidsequenzen.

**11.** Modifiziertes Plasmid, welches ausgehend von einem Plasmid gewonnen wurde, das in irgendeinem der Ansprüche 1 bis 9 definiert wurde, durch Insertion einer heterologen Nucleinsäuresequenz in dieses Plasmid, vorzugsweise einer Nucleinsäuresequenz, die für ein Peptid oder ein heterologes Protein kodiert.

**12.** Plasmid gemäß Anspruch 11, **dadurch gekennzeichnet, dass** es eine Nucleinsäuresequenz umfasst, die ausgewählt ist aus einer Nucleinsäuresequenz, die für ein Bacteriocin kodiert, einer Nucleinsäuresequenz, die für ein Immunitätsprotein gegenüber einem Bacteriocin kodiert und einer Nucleinsäuresequenz, die ein Antibiotika-Resistenzprotein kodiert.

**13.** Modifiziertes Plasmid gemäß Anspruch 11 oder Anspruch 12, **dadurch gekennzeichnet, dass** es die Sequenz SEQ ID Nr. 1 sowie eine heterologe in das Genom des Plasmids insertierte Nucleotidsequenz an einer anderen Stelle als SEQ ID Nr. 1 enthält.

14. Modifiziertes Plasmid gemäß Anspruch 11 oder Anspruch 12, **dadurch gekennzeichnet, dass** die heterologe Nucleotidsequenz in irgendeine Stelle der Sequenz SEQ ID Nr. 2 mit Ausnahme von SEQ ID Nr. 1 insertiert ist.

15. Wirtszelle, die mit einem Plasmid gemäß einem der Ansprüche 5 bis 9, oder einem modifizierten Plasmid gemäß irgendeinem der Ansprüche 12 bis 14 transformiert ist, wobei die Zelle dieses Plasmid nicht natürlicherweise umfasst.

16. Wirtszelle, umfassend ein Plasmid, welches die Sequenz SEQ ID Nr. 1 umfasst und das Gesamte oder ein Teil der Sequenz SEQ ID Nr. 2, in die eine heterologe Nucleotidsequenz inseriert wurde, ist.

17. Leuconostoc mesenteroides, umfassend das Plasmid pTLX1, welches bei der Collection Nationale de Cultures de Microorganismes (CNCM) am 30. Oktober 1997 unter der Nr. I-1936 hinterlegt worden ist.

18. Leuconostoc mesenteroides, umfassend das Plasmid pTLX1, welches bei der Collection Nationale de Cultures de Microorganismes (CNCM) am 30. Oktober 1997 unter der Nr. I-1937 hinterlegt worden ist.

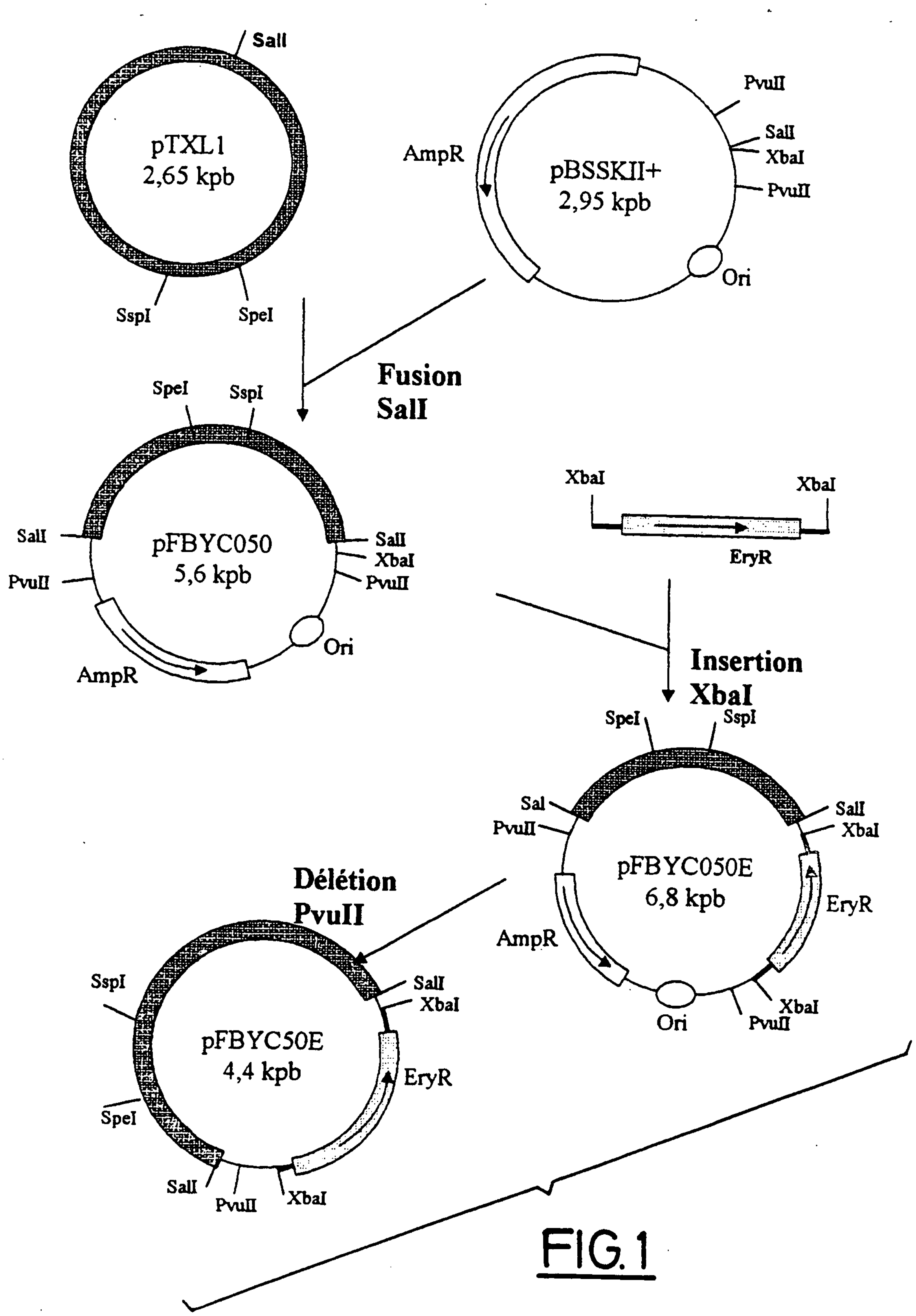
SalI
pTXL1
2,65 kpb
SspI
SpeI
AmpR
pBSSKII+
2,95 kpb
PvuII
SalI
XbaI
PvuII
Ori
Fusion
SalI
SpeI
SspI
SalI
PvuII
pFBYC050
5,6 kpb
SalI
XbaI
PvuII
Ori
AmpR
XbaI
XbaI
EryR
Insertion
XbaI
SpeI
SspI
Sal
PvuII
SalI
XbaI
pFBYC050E
6,8 kpb
EryR
AmpR
Ori
PvuII
XbaI
Délétion
PvuII
SspI
SalI
XbaI
pFBYC50E
4,4 kpb
EryR
SpeI
SalI
PvuII
XbaI

FIG.1

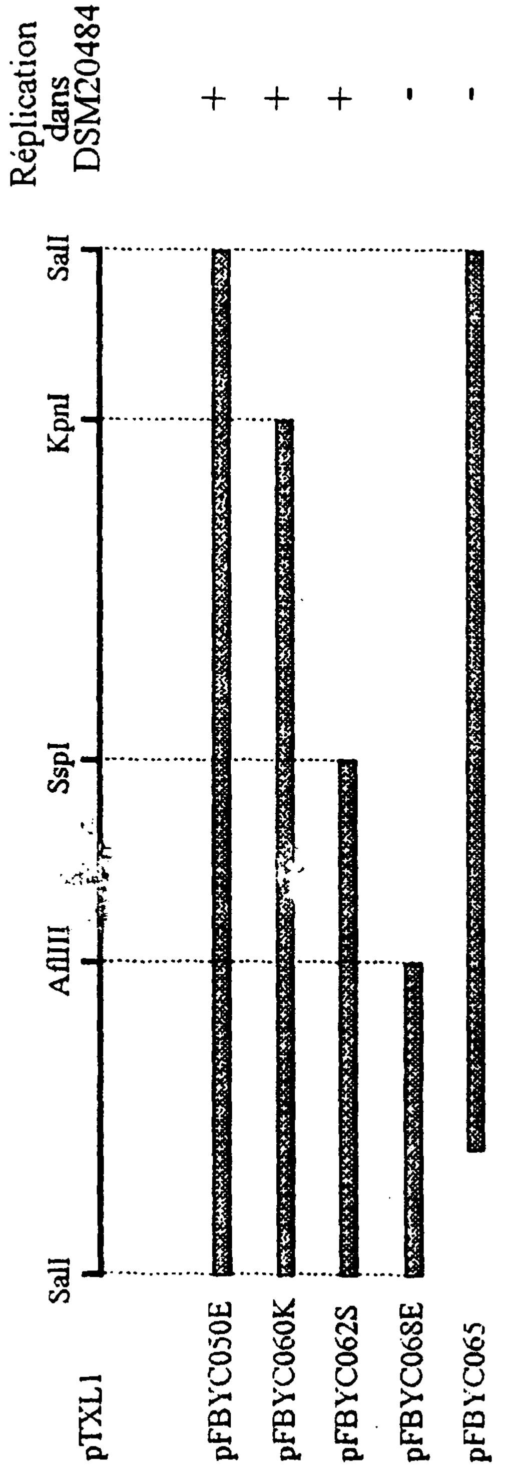
Réplication dans DSM20484
SalI
KpnI
SspI
AflIII
SalI
pTXL1
pFBYC050E
pFBYC060K
pFBYC062S
pFBYC068E
pFBYC065
+
+
+
-
-

FIG. 2

```
     SalI
   1 TCGACGTTAAAAGTTTCCATTCTGTATCTGTGAATGACTTTTTAGATAAATCTATATTAA

  61 GGCGTTTAGCTTTCTATTTAAGGCTTGACGTGATATTTCTAGTTCGTCTGCTATCGCGAT

                                                  ----------> IR1      <-.
 121 AATGGTATCAAATTCGTGTGTCATCAGTCTCTCCAAACGTAAACTGAAGTGATGTAAAGT

     ----------
 181 TTACGTTTTAAGTTTACTATATTGCTGACGTTTTAAGTAGGTCATTTAATTATTAAAACA

     ---DR1---->                ---DR1---->
 241 TAAGATTATTTGTTTGTTTATTGTCATGTATAGTTCTTAATGCTATACTCATATCAACAT

                 ↓
 301 TTAAATACAAATAAAAAGACCTCAACTCTTGCAGGAGTTAGGACTTGGTGACCTAGATAT

                                  EcoRI
 361 TAACACTATCAGGGTTTTGCCATTACAGAATTCGACCTCTGAAATGGCTTAGAATACTTA

 421 CTATTATACAAACTTATAGACTAAGAGTAAACAGCTTTACTCAAAAAAAGAACTATAAAC

          -----------> IR2      <-----------
 481 GACTATGAAAGCGTATCCTCCAGCCTAACTAAGCACGAGGATACGCTTTTTACGTCTGTT

       ----DR2----->

 541 AAGTCGTTGTCGGACGTTATCCTAACAACTAATACGGAACAGGCGTGTATCCGTCAAAGG

 601 GGCTGAAAGGTCGCTTAAACCACGTCCAAAGATACAATAGCTAACGTATCGGGGAATGAA
                                                        R  I  P  S  H  V

 661 CAATTCGATTATGGGTAGGCTCGCCCGCAAGTGATTGGCAAAGAAGTGGCATATAGAGAT
      I  R  N  H  T  P  E  G  A  L  S  Q  C  L  L  P  M  Y  L  Y

                                                           AflIII
                                                           ----DR3--->
 721 AAGCGCCTATATGGTTTAAAACGTCTGTAAGGCGATTTAAGCGGTGTCTGACGTGTTCTA
      A  G  I  H  N  L  V  D  T  L  R  N  L  R  H  R  V  H  E  L

 781 ACCTTATGATAAGGTTTTCTATTGGGCAGACGATAGAAAAGCAAATAGAGCGATATACGT
      R  I  I  L  N  E  I  P  C  V  I  S  F  C  I  S  R  Y  V  H

 841 GGTTGATACAAGCGATATGATTCTGAATTATACCTTGAACAATTTAAAAAGTCCTAAATA
      N  I  C  A  I  H  N  Q  I  I  G  Q  V  I <ORF2

 901 CTTAGGGCTTCCTCTGCTCAAATCAAACTGATTGCCCTTGTTGATTGTGATTTACATTTG

 961 GTGGTGTTATTATGAAAGCGTATATATTTCTATATCATGATATTTTAATTCTTTTTTAGA
                                                ORF1> Y  F  N  S  F  L  E

              ----DR2----->
1021 AAGGAGTCTATCTGTGACTATACTTTTTCAAGATGTTCCTGTTTCTGTTTGGGAAATCAA
      R  S  L  S  V  T  I  L  F  Q  D  V  P  V  S  V  W  E  I  N
```

FIG. 3

```
1081 TAAGAATACCCCTCAGCCCGATTGGGTTAAAAACTGTTTTGAAAATAATACTATGGTTTG
        K  N  T  P  Q  P  D  W  V  K  N  C  F  E  N  N  T  M  V  W

1141 GTATGACAATAGGTTAAAAATACTTGTAAAAGCTATCAATCCTTCTCCAAAAAGAGATGT
        Y  D  N  R  L  K  I  L  V  K  A  I  N  P  S  P  K  R  D  V

1201 TAAATTAGGTTTACGAGATACCATGTTAGGTTATTATGGTGGTGGATTTGTAATGGGTAA
        K  L  G  L  R  D  T  M  L  G  Y  Y  G  G  G  F  V  M  G  N

                                        AflIII
                                     ----DR3--->
1261 TATCGGTGATTATTTTGATGCAACAAATGGACGTGTTCTATCGAAAAAAAAGTTCTATAA
        I  G  D  Y  F  D  A  T  N  G  R  V  L  S  K  K  K  F  Y  K

                             SspI
1321 GCAATACGTTATAAACGAATAATATT
        Q  Y  V  I  N  E
```

FIG. 3 (suite)

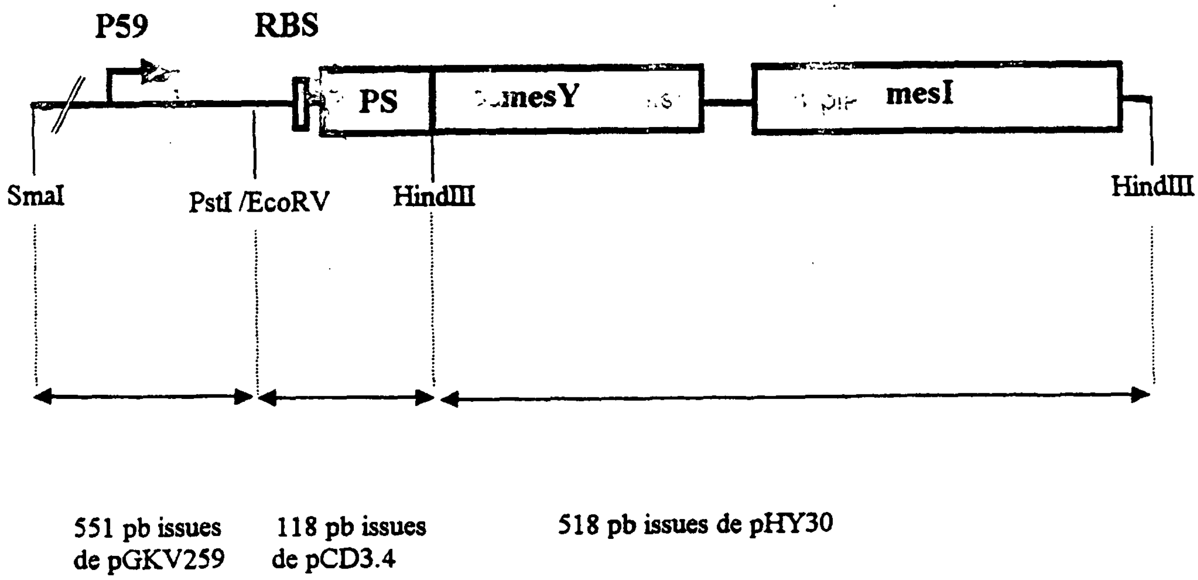


FIG. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **Benachour, A. ; J. Frère ; G. Novel.** pUCL287 plasmid from Tetragenococcus halophila (Pediococcus halophilus) ATCC 33315 represents a new theta-type repicon family of lactic acid bacteria. *FEMS Microbiology Letters,* 1995, vol. 128, 167-176
- **Berthier, F. ; M. Zagorec ; M. Champomier-Vergès ; S. D. Ehrlich ; F. Morel-Deville.** Efficient transformation of Lactobacillus sake by electroporation. *Microbiology,* 1996, vol. 142, 1273-1279
- **Biet, F. ; J.M. Berjeaud ; R. W., Worobo ; Y. Cenatiempo ; C. Fremaux.** Heterologous expression of mesentericin Y105 using the general secretion pathway (GST) or the dedicated transport sytem (DTS) in gram-positive and gram-negative bacteria. *Microbiology*
- **Fremaux, C. ; Y. Héchard ; Y. Cenatiempo.** Mesentericin Y105 gene clusters in Leuconostos mesenteroides Y105. *Microbiology,* 1995, vol. 141, 1637-1645
- **Frère, J. ; M. Novel ; G. Novel.** Molecular analysis of the Lactococcus lactis subspecies lactis CNRZ270 bidirectional theta replicating lactose plasmid pUCL22. *Molecular Microbiology,* 1993, vol. 10 (5), 1113-1124
- **Gruss, A. ; D. Ehrlich.** The family of interrelated single-stranded deoxyribonucleic acid plasmids. *MICROBIOLOGICAL REVIEWS,* Juin 1989, 231-241
- **Hayes, F. ; C. Daly ; G.F. Fitzgerald.** Identification of the minimum replicon of Lactococcus lactis subsp. lactis UC317 plasmid pCl305. *Applied and Environmental Microbiology,* 1990, vol. 56 (1), 202-209
- **Kiewiet, R. ; S. Bron ; K. De Jonge ; G. Venema ; J. F. M. L. Seegers.** Theta replication of the lactococcal plasmid pWV02. *Molecular Microbiology,* 1993, vol. 10 (2), 319-327
- **Le Chatelier, E. ; D. S. Ehrlich ; L. Jannière.** Biochemical and genetic analysis of the unidirectional theta replication of the S. agalactiae plasmid pIP501. *PLASMID,* 1993, vol. 29, 50-56
- **Leenhouts, K. J. ; B. Tolner ; S. Bron ; J. Kok ; G. Venema ; J. F. M. L. Seegers.** Nucleotide sequence and characterization of the Broad-host-range Lactococcal plasmid pWV01. *PLASMID,* 1991, vol. 26, 55-66
- **Maguin, E. ; H. Prévost ; D. S. Ehrlich ; A. Gruss.** Efficient insertioal mutagenesis in Lactococci and other Gram-positive bateria. *Journal of bacteriology,* 1996, vol. 178 (3), 931-935
- **Marugg, J. D. ; C. F. Gonzales ; B. S. Kunka ; A. M. Ledeboer ; M. J. Pucci ; M. Y. Toonen ; S. A. Walker ; L. C. Zoetmulder ; P. A. Vandenbergh.** Cloning, expression, and nucleotide sequence of genes involved in production of PA-1, a bacteriocin from Pediococcus acidilactici PAC1.0. *APPLIED AND ENVIRONMENTAL MICROBIOLOGY,* 1992, vol. 58 (8), 2360-2367
- **Muriana, P. M. ; T. R. Klaenhammer.** Cloning, phenotypic expression and DNA sequence of the gene for lactacin F, an antimicrobial peptide produced by Lactobacillus spp. *Journal of Bacteriology,* 1991, vol. 173 (5), 1779-1788
- **Raya, R. R. ; C. Fremaux ; G. L. De Antoni ; T. R. Klaenhammer.** Site-specific integration of the temperate bacteriophage fadh into the Lactobacillus gasseri chromosome and molecular characterization of the phage (attP) and bacterial(attB) attachment site. *Journal of Bacteriology,* 1992, vol. 174, 5584-5592
- Molecular cloning:a laboratory manual. Cold sping harbor laboratory, 1989
- **Sanger, F. ; S. Nicklen ; A. R. Coulson.** DNA sequencing with chain-terminating inhibitions. *Proc. Natl. Acad Sci.,* 1977, vol. 74, 5463-5467
- **Simon, D. ; A. Chopin.** Construction of a vector plasmid family and its use for molecular cloning in Streptococcus lactis. *Biochimie,* 1988, vol. 70, 559-566
- **Van Der Vossen, J. M. B. M. ; D. Van Der Lelie ; G. Venema.** Isolation and characterization of Streptococcus cremoris Wg2-specific promoters. *Applied and Environmental Microbiology,* 1987, vol. 53 (10), 2452-2457
- **Wororb, R. W. ; M. J. Van Belkum ; M. Sailer ; K. L. Roy ; J. C. Vederas ; M. E. Stiles.** A signal peptide secretion-dependent bacteriocin from Carnobacterium divregens. *Journal of Bacteriology,* 1995, vol. 177 (11), 3143-3149

LISTE DE SEQUENCES

(1) INFORMATION GENERALE:

(i) DEPOSANT:

(A) NOM: TEXEL
(B) RUE:
(C) VILLE:
(E) PAYS: FRANCE
(F) CODE POSTAL:
(G) TELEPHONE:
(H) TELECOPIE:

(ii) TITRE DE L' INVENTION: Nouveau plasmide non-RCR
(iii) NOMBRE DE SEQUENCES: 2
(iv) FORME LISIBLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.25 (OEB)

(2) INFORMATION POUR LA SEQ ID NO: 1:

(i) CARACTERISTIQUES DE LA SEQUENCE:

(A) LONGUEUR: 1346 paires de bases
(B) TYPE: acide nucléique
(C) NOMBRE DE BRINS: double
(D) CONFIGURATION: circulaire

(ii) TYPE DE MOLECULE: ADN (plasmidique)
(vi) ORIGINE:

(A) ORGANISME: Leuconostoc mesenteroides

(xi) DESCRIPTION DE LA SEQUENCE: SEQ ID NO: 1:

```
TCGACGTTAA AAGTTTCCAT TCTGTATCTG TGAATGACTT TTTAGATAAA TCTATATTAA     60
GGCGTTTAGC TTTCTATTTA AGGCTTGACG TGATATTTCT AGTTCGTCTG CTATCGCGAT    120
AATGGTATCA AATTCGTGTG TCATCAGTCT CTCCAAACGT AAACTGAAGT GATGTAAAGT    180
TTACGTTTTA AGTTTACTAT ATTGCTGACG TTTTAAGTAG GTCATTTAAT TATTAAAACA    240
TAAGATTATT TGTTTGTTTA TTGTCATGTA TAGTTCTTAA TGCTATACTC ATATCAACAT    300
TTAAATACAA ATAAAAAGAC CTCAACTCTT GCAGGAGTTA GGACTTGGTG ACCTAGATAT    360
TAACACTATC AGGGTTTTGC CATTACAGAA TTCGACCTCT GAAATGGCTT AGAATACTTA    420
CTATTATACA AACTTATAGA CTAAGAGTAA ACAGCTTTAC TCAAAAAAAG AACTATAAAC    480
GACTATGAAA GCGTATCCTC CAGCCTAACT AAGCACGAGG ATACGCTTTT TACGTCTGTT    540
AAGTCGTTGT CGGACGTTAT CCTAACAACT AATACGGAAC AGGCGTGTAT CCGTCAAAGG    600
GGCTGAAAGG TCGCTTAAAC CACGTCCAAA GATACAATAG CTAACGTATC GGGGAATGAA    660
CAATTCGATT ATGGGTAGGC TCGCCCGCAA GTGATTGGCA AAGAAGTGGC ATATAGAGAT    720
AAGCGCCTAT ATGGTTTAAA ACGTCTGTAA GGCGATTTAA GCGGTGTCTG ACGTGTTCTA    780
```

```
ACCTTATGAT AAGGTTTTCT ATTGGGCAGA CGATAGAAAA GCAAATAGAG CGATATACGT    840
GGTTGATACA AGCGATATGA TTCTGAATTA TACCTTGAAC AATTTAAAAA GTCCTAAATA    900
CTTAGGGCTT CCTCTGCTCA AATCAAACTG ATTGCCCTTG TTGATTGTGA TTTACATTTG    960
GTGGTGTTAT TATGAAAGCG TATATATTTC TATATCATGA TATTTTAATT CTTTTTTAGA   1020
AAGGAGTCTA TCTGTGACTA TACTTTTTCA AGATGTTCCT GTTTCTGTTT GGGAAATCAA   1080
TAAGAATACC CCTCAGCCCG ATTGGGTTAA AAACTGTTTT GAAAATAATA CTATGGTTTG   1140
GTATGACAAT AGGTTAAAAA TACTTGTAAA AGCTATCAAT CCTTCTCCAA AAAGAGATGT   1200
TAAATTAGGT TTACGAGATA CCATGTTAGG TTATTATGGT GGTGGATTTG TAATGGGTAA   1260
TATCGGTGAT TATTTTGATG CAACAAATGG ACGTGTTCTA TCGAAAAAAA AGTTCTATAA   1320
GCAATACGTT ATAAACGAAT AATATT                                        1346
```

(2) INFORMATION POUR LA SEQ ID NO: 2:

(i) CARACTERISTIQUES DE LA SEQUENCE:

(A) LONGUEUR: 2665 paires de bases
(B) TYPE: acide nucléique
(C) NOMBRE DE BRINS: double
(D) CONFIGURATION: circulaire

(ii) TYPE DE MOLECULE: ADN (plasmidique)
(vi) ORIGINE:

(A) ORGANISME: Leuconostoc mesenteroides

(xi) DESCRIPTION DE LA SEQUENCE: SEQ ID NO: 2:

```
TCGACGTTAA AAGTTTCCAT TCTGTATCTG TGAATGACTT TTTAGATAAA TCTATATTAA    60
GGCGTTTAGC TTTCTATTTA AGGCTTGACG TGATATTTCT AGTTCGTCTG CTATCGCGAT   120
AATGGTATCA AATTCGTGTG TCATCAGTCT CTCCAAACGT AAACTGAAGT GATGTAAAGT   180
TTACGTTTTA AGTTTACTAT ATTGCTGACG TTTTAAGTAG GTCATTTAAT TATTAAAACA   240
TAAGATTATT TGTTTGTTTA TTGTCATGTA TAGTTCTTAA TGCTATACTC ATATCAACAT   300
TTAAATACAA ATAAAAAGAC CTCAACTCTT GCAGGAGTTA GGACTTGGTG ACCTAGATAT   360
TAACACTATC AGGGTTTTGC CATTACAGAA TTCGACCTCT GAAATGGCTT AGAATACTTA   420
CTATTATACA AACTTATAGA CTAAGAGTAA ACAGCTTTAC TCAAAAAAAG AACTATAAAC   480
GACTATGAAA GCGTATCCTC CAGCCTAACT AAGCACGAGG ATACGCTTTT TACGTCTGTT   540
AAGTCGTTGT CGGACGTTAT CCTAACAACT AATACGGAAC AGGCGTGTAT CCGTCAAAGG   600
GGCTGAAAGG TCGCTTAAAC CACGTCCAAA GATACAATAG CTAACGTATC GGGGAATGAA   660
CAATTCGATT ATGGGTAGGC TCGCCCGCAA GTGATTGGCA AAGAAGTGGC ATATAGAGAT   720
AAGCGCCTAT ATGGTTTAAA ACGTCTGTAA GGCGATTTAA GCGGTGTCTG ACGTGTTCTA   780
ACCTTATGAT AAGGTTTTCT ATTGGGCAGA CGATAGAAAA GCAAATAGAG CGATATACGT   840
GGTTGATACA AGCGATATGA TTCTGAATTA TACCTTGAAC AATTTAAAAA GTCCTAAATA   900
```

```
CTTAGGGCTT CCTCTGCTCA AATCAAACTG ATTGCCCTTG TTGATTGTGA TTTACATTTG    960
GTGGTGTTAT TATGAAAGCG TATATATTTC TATATCATGA TATTTTAATT CTTTTTTAGA   1020
AAGGAGTCTA TCTGTGACTA TACTTTTTCA AGATGTTCCT GTTTCTGTTT GGGAAATCAA   1080
TAAGAATACC CCTCAGCCCG ATTGGGTTAA AAACTGTTTT GAAAATAATA CTATGGTTTG   1140
GTATGACAAT AGGTTAAAAA TACTTGTAAA AGCTATCAAT CCTTCTCCAA AAAGAGATGT   1200
TAAATTAGGT TTACGAGATA CCATGTTAGG TTATTATGGT GGTGGATTTG TAATGGGTAA   1260
TATCGGTGAT TATTTTGATG CAACAAATGG ACGTGTTCTA TCGAAAAAAA AGTTCTATAA   1320
GCAATACGTT ATAAACGAAT AATATTAAAT TATGACGTTC TATATTTTTG TCTGATGAGG   1380
AATGTGAAAA TGTTGTTGGA GGGCATTGGT TATCAGATCT TCTTGGTGGG ATTGCACATA   1440
CATATCATCC TGCTGACCCC CAAAGAGTTC TTGACCAATT AAATGGCAAG ACTCAACCTA   1500
AACCAGGGCA TCAATGCAGC CCTTATGGTT ACTATTAAAC ATTCAGTTAA CATAAGGGTC   1560
ATTATACAAA GTAAAAAGCG ATAAACCACT TCTAGCAAGG GTTTATCGCT TTTTGAGTAC   1620
CAACTAGTTT GTGAATTCGT CTTTTTTTGA TTGGTCTAGC TGGTTAAACC TTGGTTTAAT   1680
CACGTTGCTA GTTTGTGAAA TGACTAGATT TTAAGCACGC TTTTTTCTGG ATACTTGTAA   1740
GAATATCAGA TATGAAGCCG TAAAAACTAC AAAGTAGACA ACATCAAACC AAAATGATGT   1800
ACTTAATGTA CCACCGTCTA ACAGTTGAAA CACAATATCA ATAGCACACC AACCAGCAAT   1860
AGTTATCCCT ATGCTAATCA TTAATTTAAC GAACCAATTC ATTAGTAATC GCCCTCTGTT   1920
TCTATTGTAT CAACAACCAA CATTGTTGAG TAGCTAAATT GGTATCTAAT CTAATTATAC   1980
TAATAAGCAA AATAATCCGT CAGCAAAACG ACATAAAATG AGTTTAATGC GCACTTACAC   2040
ATCACTTTTT CAAGTGATGA AATTGCTTGA ACATTTTATC AGAATGCGCA CTTACACACC   2100
ACACATAAAT TGGTGGTGGT ATTGCCCAAA AAAATTGTGT CAGAAGTCGC TTATAGCGAC   2160
AACTTAAAAA CCAACAAAAG TTCTTGTTGC GAGCATACTA AGTGGGTACC CACTTAGGGT   2220
AAAAACTAAT CATCTGACGA GCTAAACAAC CTAAAATGTA ATTTTAGATT GCCGCTCTTT   2280
CAGATGATTA GTTTTTGTTT TTGGGGTTTC CCAATCCCTT TTTGAATTAA AATTAATTTT   2340
GATAAGCGGA CGGTTGATGA ACGAAAAATA CTAACAAACA AAACGGACGC TATAAAATTA   2400
CCTTTGAAAA ACTATTTAAA TAATCTATGC CAGAATCCTT TCTTTGGCTG ATTTTCTTTT   2460
TCTTGATACT CAATTAGGGT TTTATTTGTC TCTGTTAATC TTTGTTCAGC AATTAATTGC   2520
AATTGTTGCG CATGATCAAT TTGTTTATCT TTTTCTTTAA TTTGCGATTT TAAATAATTA   2580
ATTAAATCAT CTTTTTCAGC TAGTTGTTGC GCTGTAAAAG TGTCAACATA GTTACTTGAA   2640
GTTGACTGTT TTGGTTTACG TTTAG                                          2665
```